# EUROPEAN PATENT APPLICATION

(11) **EP 4 591 780 A1**
(43) Date of publication of application: **30.07.2025**
(21) Application number: 25153253.7
(22) Date of filing: 22.01.2025
(51) Int. Cl.: A61B 1/00, A61B 1/015, A61L 2/26, A61M 39/12

(54) **SYSTEM AND METHOD FOR CONNECTING A MEDICAL ACCESSORY TO AN ENDOSCOPE**

(30) Priority: 26.01.2024 NL 2036899
(71) Applicant: Wassenburg Medical B.V., 6669 NA Dodewaard (NL)
(72) Inventor: HOFENK, Stefan Willeon, 6515 EM Nijmegen (NL); MEULENBROEK, Gerardus Jacobus Johannes, 6922 GD Duiven (NL)
(74) Representative: Patentwerk B.V.

(57) **Abstract**

A system and a method for connecting a medical accessory to an endoscope, wherein the system comprises the medical accessory and wherein the medical accessory comprises a protruding element and a slide. The slide is movable, relative to the medical accessory between a first position and second position.

## Description

The present invention relates to a system and a method for connecting a medical accessory to an endoscope.

Systems for connecting a medical accessory, such as a channel separator, to endoscopes are known in the art. Such systems are typically used during the decontamination, i.e. cleaning and disinfecting, (of the channels) of the endoscope. For example, an endoscope typically includes a plurality of channels which can be configured to direct water, air, and/or any other suitable fluid into a surgical site. In some circumstances, one or more channels in an endoscope can be configured to guide a surgical instrument into the surgical site.

Decontamination systems can be used to reprocess previously-used medical devices, such as endoscopes, such that the devices can be used once again. During the decontamination process of some endoscopes, for example the flow in the air channel(s) and/or water channel(s) within the endoscope can be measured to verify that the channels are unobstructed. Also, a source of fluid can be attached to the channel inlets of the endoscope such that fluid from a fluid source can flow through the channels. In order to provide a controllable fluid flow through the channels, systems for connecting a medical accessory to an endoscope, such as channel separators are used to separate the channels of a medical device. Such a system may for example be provided to avoid a flow of fluid through a channel configured to direct air into a surgical site.

Known systems for connecting a medical accessory, such as a channel separator, to endoscopes have a body with inserts for medical accessories. The known systems for example comprise one or multiple springs to lock and unlock the medical accessories to and from the endoscope, respectively. To lock the body on the endoscope, the user needs to exert a force on the body which overcomes the spring constant(s) of the one or multiple springs. The known systems are therefore relatively user-unfriendly and heavy to operate.

For example, US 2017 007 357 A1 discloses a connecting device for coupling a rinsing system to a channel of a surgical instrument to be cleaned, a cleaning and/or disinfecting device and method for the operation thereof.

The present invention aims to overcome the abovementioned drawbacks. Thereto is provided a system for connecting a medical accessory to an endoscope, wherein the system comprises the medical accessory comprising at least one protruding element, and a slide. The slide comprises an upper side and a lower side, and at least one passage opening extending between the upper side and the lower side of the slide configured for passaging at least a part of the medical accessory, wherein the slide is movable relative to the medical accessory between a first position and second position, wherein in the first position, the medical accessory is positioned in the wider part of the at least one passage opening at the upper side of the slide, and at least a part of the endoscope to be connected to the medical accessory is positioned in a broader portion of the at least one passage opening at the lower side of the slide, and wherein in the second position, the medical accessory is positioned at least partially beyond the narrower part of the passage opening at the upper side of the slide, and at least a part of the endoscope is positioned in a slimmer portion of the at least one passage opening at the lower side of the slide to limit movement between the slide and the endoscope.

The invention in particular proposes a system according to claim 1. The slide is moveable between the first and second position, preferably to lock and unlock the medical accessory to or from the endoscope. The narrower part of the passage opening at the upper side of the slide may be configured to exert a locking force on the medical accessory, preferably to keep the medical accessory in a locking configuration or in the second position. The slimmer portion of the passage opening at the lower side of the slide may be configured to exert a locking force on at least a part of the endoscope, preferably to keep the slide and/or the medical accessory in a locking configuration or in the second position. The endoscope to connect the medical accessory to may be provided with at least one port to connect the medical accessory to, the port for example being a suction valve, an air/water valve or an instrument channel of the endoscope. The slimmer portion of the passage opening at the lower side of the slide may be configured to exert a locking force on the port the endoscope, preferably to keep the slide and/or the medical accessory in a locking configuration or in the second position. The narrower part may be a transition that needs to be overcome to move the components from the wider part towards another section of the slide, where the components may be locked. In general, the at least one passage opening of the slide comprises a wider part and a broader portion for allowing entry of the components and a narrower part and a slimmer portion to lock the components with the endoscope. The slide is positioned in the first position when the medical accessory is positioned in the wider part of the passage opening at the upper side of the slide and when the medical accessory is positioned in the broader portion of the passage opening at the lower side of the slide. The medical accessory is preferably at least partially inserted into at least one port of the endoscope in the first position, preferably until the at least one protruding element abuts the slide and/or the at least one port of the endoscope. The slide is positioned in the second position when the medical accessory is positioned at least partially beyond the narrower part of the passage opening at the upper side of the slide and when the medical accessory is positioned in the slimmer portion of the passage opening at the lower side of the slide. It is conceivable that the medical accessory is provided beyond the narrower part of the passage opening at the upper side of the slide in a direction away from the first position and/or at a distant location from the first position. It is imaginable that the medical accessory is provided beyond the slimmer portion of the passage opening at the lower side of the slide in a direction away from the first position and/or at a distant location from the first position. To allow movement of the slide and/or the medical accessory relatively to each other, the second position and the first position are preferably located at a distance from each other. The slide may be positioned in the second position when the medical accessory is positioned at least partially in the narrower part of the passage opening at the upper side of the slide and/or when the medical accessory is positioned entirely beyond the narrower part of the passage opening at the upper side of the slide. The slide may be positioned in the second position when the medical accessory is positioned at least partially in the slimmer portion of the passage opening at the lower side of the slide. The wider and the narrower part of the passage opening at the upper side of the slide may for instance be two opposition parts of the passage opening. It is not essential that the wider part is the widest part and the narrower part is the narrowest part, however, it may be possible that a transition between the wider and the narrower part comprises a portion narrower than both, for instance forming sort of a barrier or resistance that needs to be overcome before transitioning between the two parts of the passage opening. The broader and the slimmer portion of the passage opening at the lower side of the slide may for instance be two opposition parts of the passage opening. It is not essential that the broader portion is the broadest portion and the slimmer portion is the slimmest portion, however, it may be possible that a transition between the broader and the slimmer portion comprises a portion slimmer than both, for instance forming a barrier or resistance that needs to be overcome before transitioning between the two portions of the passage opening. Any reference to an endoscope in the system is purely to elaborate on its purpose and functionality, the system itself does not comprise the endoscope.

The at least one passage opening at the lower side of the slide may at least partially taper along a slide direction, wherein the slide direction extends between the first position and the second position of the slide. Preferably, the slide direction at least extends from the wider part to the narrower part of the at least one passage opening at the upper side of the slide and/or from the broader portion and the slimmer portion of the passage opening at the lower side of the slide. In the first position, the broader portion of the passage opening at the lower side of the slide may relatively easily be placed on the endoscope, in particular around at least one port of the endoscope. In the second position, the slimmer portion of the passage opening at the lower side of the slide is configured to connect to at least a part of the endoscope to limit movement between the slide and the endoscope. Preferably, the slimmer portion of the passage opening at the lower side of the slide is configured to connect to at least one port of the endoscope to limit movement between the slide and the port of the endoscope.

The wider part of the at least one passage opening at the upper side of the slide may at least partially align with at least a part of the broader portion of the tapered or asymmetric shape of the at least one passage opening at the lower side of the slide to allow insertion of the medical accessory and/or to allow the insertion of at least a part of the endoscope, in particular at least a part of the port of the endoscope, in the passage opening of the slide. Optionally, the broader portion aligns with a widest part of the passage opening or a broadest portion aligns with the wider part of the passage opening or the broadest portion aligns with the widest part of the passage opening. The narrower part of the at least one passage opening at the upper side of the slide may at least partially align with at least a part of the slimmer portion of the tapered or asymmetric shape of the at least one passage opening at the lower side of the slide to simultaneously limit movement between the slide and the endoscope and to limit movement between the slide and the medical accessory. It is imaginable, that the slimmer portion of the passage opening at the lower side of the slide and the narrower portion or a narrowest part of the passage opening at the upper side of the slide are shifted relatively to each other, preferably such that in the second position the medical accessory is positioned at least partially beyond the narrower part and in the slimmer portion of the passage opening.

In a preferred embodiment of the invention, the width of the at least one passage opening at the upper side of the slide is smaller than the width of the at least one protruding element of the medical accessory to limit movement between the slide and the medical accessory. It may also be preferred that, at the lower side of the slide, the at least one passage opening has a tapered or asymmetric shape. Additionally, it may be preferred that, at the upper side of the slide, the at least one passage opening comprises a wider part and a narrower part. It may be preferred that the slide is movable, in particular slidable, relative to the medical accessory between a first position and second position. In the context of the present invention, this means that the slide may slide from a first position to a second position, relative to the medical accessory.

The width of the slimmer portion of the at least one passage opening of the slide at the lower side of the slide may be smaller than a flanged portion of at least one port of the endoscope to limit movement between the slide and the port of the endoscope and/or to lock the slide and the endoscope in the second position. A lower side of the flanged portion of the endoscope may be in contact with the lower side of the passage opening, preferably at least in the second position to improve the limitation of movement between the slide and the port of the endoscope. The width of the broader portion of the passage opening at the lower side of the slide is preferably larger than the flanged portion of the port of the endoscope to easily pass at least a part of the port, in particular the flanged portion, through the passage opening in the first position.

The width of the at least one passage opening at the upper side of the slide may comprise a narrowest part. Preferably, the narrowest part is arranged between two side ends of the passage opening. The width of the passage opening at the upper side of the slide may for instance have a wider part at one end and a narrow part at the opposite end. The narrowest part may be located between the wider part and the narrower part of the passage opening. It is imaginable that the passage opening is eight-shaped. By providing a narrowest part in between the two ends, at a predetermined position, a feedback or click system may be provided indicating that the slide has reached one of its positions (the first position and/or the second position). If for example the narrowest part is arranged towards the narrower part, the narrowest part may be passed just before the slide enters its end position. Passing the narrowest part may provide tactile feedback in the form of a click or a slight release in force, indicating to the user that the end position in reached.

The medical accessory may be substantially oblong. Preferably, the medical accessory is longer than it is wide. The dimensions of the medical accessory may be adjusted to the dimensions of a port of an endoscope to allow insertion of the medical accessory in the port of the endoscope. In a preferred embodiment the slide mainly extends in a direction perpendicular to the longitudinal direction of the medical accessory.

The at least one protruding element of the medical accessory may at least partially protrude in a substantially perpendicular direction to a longitudinal direction of the medical accessory. The at least one protruding element may for example extend in a direction perpendicular to the longitudinal direction of the medical accessory. It is imaginable that the protruding element is a flange or flange shaped. In a preferred embodiment the protruding element extends in a direction parallel to the longitudinal direction of the slide. The width of the at least one passage opening at the upper side of the slide is smaller than the width of the at least one protruding element of the medical accessory to limit movement between the slide and the medical accessory, in particular in an upwardly or vertical direction, more in particular in a perpendicular direction of the longitudinal direction of the slide.

Preferably, the at least one protruding element is configured to be at least partially in contact with and/or to at least partially abut the upper side of the slide to limit (vertical) movement between the slide and the medical accessory. Optionally, the at least one protruding element is configured to exert a substantially vertical or downwardly directed force on the slide to at least partially (temporarily) connect the slide and the medical accessory.

The medical accessory may comprise at least one second protruding element located below the at least one protruding element, preferably wherein the second protruding element is configured to be at least partially in contact with and/or to at least partially abut the lower side of the slide to limit movement between the slide and the medical accessory. The second protruding element may be a flange or be flanged shaped. It is imaginable that the second protruding element is provided in the passage opening between the upper side and the lower side of the slide to improve the connection of the system with the endoscope. Preferably, the dimensions of the second protruding element are such that it can entirely be received in the passage opening of the slide. The second protruding element may be at least partially in contact with at least one port of the endoscope to further limit movement between the slide and the medical accessory. The dimensions of the second protruding element are preferably such that the second protruding element can at least be partially be received in the passage opening of the slide. It is imaginable that the dimensions of the second protruding element are adjustable, preferably such that the system may be connected to different ports of the endoscope and/or to different endoscopes. In particular, the dimensions of the second protruding element are such that it can at least be partially received in the first position of the slide.

The medical accessory may comprise at least two ends located on opposite sides of the medical accessory. Preferably, the two ends are located at longitudinal end points of the medical accessory. The at least one protruding element may be provided between the at least two ends. Preferably, the second protruding element is provided between the at least two ends.

At least one end may be a closed end, preferably wherein the at least two ends are closed ends, more preferably wherein all (or both) the ends are closed ends to block and/or to seal at least one port of the endoscope, in particular to block and/or seal at least the access to at least one port of the endoscope and/or to block and/or seal at least the exit of at least one port of the endoscope. It is imaginable that the at least one protruding element is provided on at least one end to close said end.

It is imaginable that the medical accessory comprises at least two ends located on opposite sides of the medical accessory, wherein at least one end is an open end, preferably wherein the at least two ends are connected open ends for allowing passaging of fluid at least between a first open end and a second open end of the medical accessory. The medical accessory may be tube shaped comprising two open ends which are connected by a hollow tube portion configured to conduct fluid from a first end to a second end of the medical accessory, in particular to conduct fluid from the first end located above the at least one protruding element to the second end located below the at least one protruding element. A second protruding element may be provided between the two ends. The second end may be inserted in a port of the endoscope. The fluid may be conducted from the first end of the medical accessory to the second end of the medical accessory into the port of the endoscope, for example to flush the ports of the endoscope. The first open end may be located above the at least one protruding element and/or the second protruding element and the second open end may be located below the at least one protruding element and/or the second protruding element.

The medical accessory may comprise a resistance element, such as barbs, at an open end of the medical accessory, preferably at the (first) open end located above the protruding element of the medical accessory, for holding a flexible fluid tube, such as for holding a flexible cleaning tube. The flexible fluid tube may be a flexible cleaning tube. The (first) open end of the medical accessory may be used to couple the system to a fluid source, such as a cleaning device. The connection between the medical accessory and the fluid source, such as the cleaning device, may be a flexible transparent hose for instance, which can be easily connected to the medical accessory by providing barbs, in particular hose barbs, on the (first) open end of the medical accessory. It is imaginable that the flexible fluid tube is permanently connected to the medical accessory and/or detachable connected to the fluid source to provide a relatively easy and robust coupling of the fluid source with the medical accessory. The term "fluid" may for example refer to a medium in liquid, gas and/or vapour phase or combination.

The medical accessory may comprise an annular recess at a lower side of the at least one protruding element for accommodating an O-ring configured to provide a seal in a space between at least a part of the medical accessory to be connected to at least a part of the endoscope and the endoscope, in particular between at least a part of the medical accessory to be inserted in at least one port of the endoscope and the port of the endoscope. Preferably, the annular recess is provided at or close to an end (or the second end) of the medical accessory at the lower side of the at least one protruding element. In order to improve the seal between the endoscope and the system, an O-ring may be used. O-rings are well known in the art and have been used to improve seals for a long time, so it's function is considered to be known in the art.

The medical accessory may comprise at least two annular recesses at a lower side of the protruding element for accommodating two O-rings configured to provide separation between channels, such as a water channel and/or an air channel, in an endoscope. Preferably, the annular recesses are provided at or close to an end (or the second end) of the medical accessory at the lower side of the at least one protruding element. The O-rings may at least partially be composed of silicone and/or viton.

The medical accessory may have different functionalities. In an example, the medical accessory acts as a stop, blocking flow of a fluid into or from the endoscope port. In that case, the stop is connected to the port and blocks any flow. In another example the medical accessory acts as a flow-through channel and has an entry for fluid and an exit for fluid, allowing to feed the fluid to or from the port. In another example the medical accessory acts as a channel separator, separating different channels located inside the endoscope port, for example to allow selective or separate operations to be applied to the different channels and keep the channels separated, for example for hygienic purposes. To that end the medical accessory may comprise a separation element such as an O-ring, to block fluid connection between the channels in the endoscope.

The medical accessory may be anything or any component configured to be connected to an endoscope, in particular to be connected to at least one port of the endoscope. The medical accessory may for example be an air/water valve insert, an instrument channel insert, a biopsy valve insert, a suction valve insert, a sealing valve insert, a gas insufflation valve insert, and the like. It will be apparent that the invention is not limited to the named examples for a medical accessory, but that numerous variants are possible.

The slide may comprise at least one at least partially deformable space located next to at least a part of the at least one passage opening at least at the upper side of the slide, wherein the deformable space is configured to at least partially deform upon the relative movement of the slide and the medical accessory between the first and second position. Preferably, the deformable space is located next to at least a part of at least one side of the perimeter of the passage opening. The deformable space may be located next to at least a part of the narrower part of the passage opening, in particular next to at least a part of the narrowest part of the passage opening. The at least one at least partially deformable space may extend between the upper side of the slide and the lower side of the slide, preferably to increase the volume allowing deformation. Therewith, the compressing force exerted on the slide may be distributed over a larger deformable area. It is conceivable that the deformable space is resilient. The deformable space may be parallel to at least a part of at least one side of the passage opening. It is imaginable that an at least partially deformable space is at least partially located between the at least two passage openings of the slide. The deformable space enables the material of the slide, in particular the perimeter of the passage opening, to deform upon the relative movement of the slide and the medical accessory between the first position and the second position. In particular, the deformable space enables the material of the slide, in particular the perimeter of the passage opening, to deform elastically upon the relative movement of the slide and the medical accessory between the first position and the second position. Therewith, the deformable space enables a reduction of the pressure applied on the material of the slide and/or of the medical accessory. Resulting in a slide and/or a medical accessory that is less prone to breakage, wear and/or fractures.

The height of the slide, or the distance between the upper and the lower side of the slide, may be approximately or substantially be the same as the combined thickness of the protruding element of the medical accessory and a flange-like portion of the port of the endoscope the medical accessory is connected to. This allows for a snug connecting, securing the portions between the sides of the slide.

The system may comprise at least two medical accessories, wherein the slide comprises at least two passage openings for passaging the medical accessories. The passage openings on the slide define the location for passaging of the medical accessories. The configuration of the ports and/or the channels of the endoscope may define the configuration of the slide, in particular the location of the passage openings. When the medical accessories are connected to the endoscope, they may be arranged at a predetermined position, for instance depending on the mutual location of the passage openings. This allows the medical accessories to be coupled to two separate ports of the endoscope for instance. It is imaginable that different medical accessories or similar medical accessories are provided in the respective passage opening of the slide. For example, a medical accessory comprising two closed ends may be provided in a first passage opening to block and/or seal the respective port of the endoscope, wherein in a second passage opening a medical accessory comprising two open ends is provided to allow fluid conduction to the port and/or channel of the endoscope. The described examples are for illustrative purposes only, since other combinations are imaginable.

The slide may be arranged to move, more in particular to slide, in direction perpendicular relative to a longitudinal direction of the medical accessory. The medical accessory may for instance be positioned mainly vertical or upright and the slide may then be positioned mainly horizontal, to facilitate coupling and allow the most efficient force transfer on the elements during coupling. Optionally, when the system is connected to the endoscope, the slide may be arranged to move, more in particular to slide, in direction perpendicular relative to a longitudinal direction of at least one port of the endoscope.

The slide may comprise a grip or a gripping portion for gripping the slide and exerting a force on the slide. In particular when the passage opening(s) at the upper side of the slide has/have a narrowest part in between their extremes or two side ends of the passage opening, a force needs to be overcome when pushing or sliding the slide between its positions. In order to comfortably exert this force a grip may be provided on the slide.

It is imaginable that the slide is at least partially composed of a polymer material, in particular a thermoplastic polymer material, such as polypropylene or polyamide to allow (slight) deformation of the slide, in particular the passage opening(s), upon movement of the slide relative to the medical accessory between the first and second position. It is imaginable that the slide is entirely composed of a polymer material, in particular a thermoplastic polymer material. The slide may at least be partially composed of a plastic material. Preferably, the material of the slide is sterilizable such that the slide may be reused.

The medical accessory may at least be partially composed of a metal, in particular of steel, more in particular of stainless steel to improve robustness of the medical accessory. It is imaginable that the medical accessory is entirely composed of a metal, in particular steel, more in particular of stainless steel. Preferably, the material of the medical accessory is sterilizable such that the medical accessory may be reused. Preferably, at least one component of the system, such as the medical accessory, is at least partially composed of metal and at least one other component of the system, such as the slide, is at least partially composed of a polymer material to provide a smooth transition between the first and second position. In another embodiment, the slide may be at least partially composed of a metal, such as stainless steel, and the medical accessory is at least partially composed of a (thermoplastic) polymer material.

In the context of the present invention, a slide for use in a system according to the present invention is disclosed.

In the context of the invention, it is also disclosed a combination of a system according to the present invention and an endoscope, wherein the endoscope comprises at least one port to connect the medical accessory to, the port for example being a suction valve, an air/water valve or an instrument channel of the endoscope.

It is imaginable that the at least one port of the endoscope comprises a flanged portion, and wherein the at least one passage opening of the slide at the lower side of the slide is configured receive at least the flanged portion of the at least one port of the endoscope. A lower side of the flanged portion of the endoscope may be in contact with the lower side of the passage opening, preferably at least in the second position to limit movement between the slide and the port of the endoscope. The flanged portion may hook in at least a part of the passage opening of the slide, preferably at a location between the upper side and the lower side of the slide. The flanged portion may hook in at least a part of the passage opening of the slide, preferably at a location between the upper side and the lower side of the slide. It is imaginable that medical accessory comprises a second protruding element below the first protruding element which is configured to be at least partially in contact with the flanged portion of the port to further limit movement between the slide and the medical accessory.

The invention further relates to a method for connecting a medical accessory to an endoscope. The method comprises the steps of
a) providing a medical accessory comprising at least one protruding element,
b) providing a slide, comprising: an upper side and a lower side, and at least one passage opening extending between the upper side and the lower side of the slide configured for passaging at least a part of the medical accessory,
c) passaging the medical accessory at least partially through the wider part of the at least one passage opening at the upper side of the slide,
d) passaging at least a part of a port of an endoscope at least partially through the broader portion of the at least one passage opening at the lower side of the slide,
e) moving the slide relative to the medical accessory and/or the port of the endoscope, such that the medical accessory is positioned at least partially beyond the narrower part of the passage opening at the upper side of the slide and such that at least a part of the port of the endoscope is positioned in a slimmer portion of the at least one passage opening at the lower side of the slide.

According to the present invention, step c) comprises the step of passaging the medical accessory at least partially through a wider part of the at least one passage opening at the upper side of the slide. Preferably, step c) ends, when the at least one protruding element is in contact with the upper side of the slide and/or abuts the upper side of the slide.

In an embodiment of the invention, it is preferred that the width of the at least one passage opening at the upper side of the slide is smaller than the width of the at least one protruding element of the medical accessory to limit movement between the slide and the medical accessory. Additionally, it may be preferred that, at the lower side of the slide, the at least one passage opening has a tapered or asymmetric shape. In the context of the present invention, it may also be preferred that, at the upper side of the slide, the at least one passage opening comprises a wider part and a narrower part. The slide may may or slide between a first position and a second position relative to the medical accessory and/or the endoscope, preferably the port of the endoscope. In the context of the present invention, it may be preferred that at least a part of a port of an endoscope passages at least partially through the broader portion of the at least one passage opening at the lower side of the slide. Preferably, the at least a part of a port of an endoscope can be formed from or comprise a flanged portion of the port of the endoscope.

It is particularly preferred that the method is carried out by using a system according to the present invention.

The invention will be further elucidated by several examples and with reference to the appended figures, wherein
- figure 1 shows an exploded view of an embodiment of the system according to the present invention;
- figure 2 shows a cross-sectional view of an embodiment of the system according to the present invention connected to an endoscope;
- figure 3 shows a top view of an embodiment of the system according to the present invention; and
- figures 4a and 4b show a bottom view of an embodiment of the system according to the present invention.

Within these figures, similar reference numbers correspond to similar or equivalent elements or features.

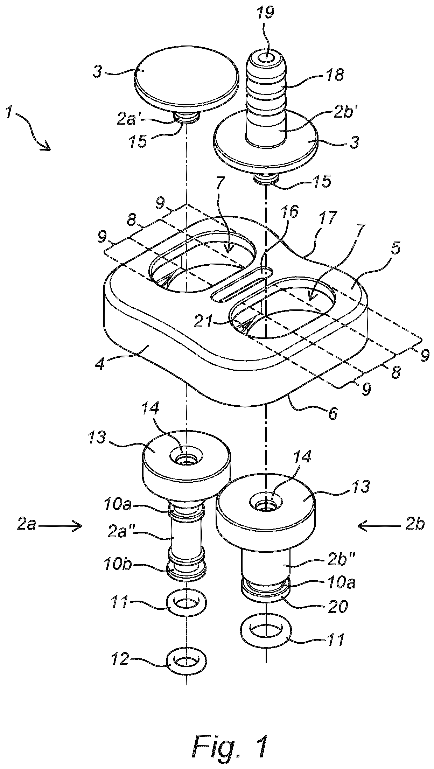

Figure 1 shows an exploded view of an embodiment of the system 1 according to the present invention. The system 1 comprises a multitude of elements. First, the system 1 comprises two medical accessories 2a, 2b each comprising a protruding element 3, preferably a flange-shaped protruding element 3. The shown medical accessories 2a, 2b are substantially oblong or elongated, however other shaped medical accessories are imaginable. The medical accessories 2a, 2b each comprise two connectable accessory parts 2a', 2a"; 2b', 2b", respectively. A first accessory part 2a', 2b' comprises a first coupling element 15. Here, the first coupling element 15 is located below the protruding element 3. The second accessory part 2a", 2b" comprises a second coupling element 14 complementary to the first coupling element 15. The first coupling element 15 and the second coupling element 14 are configured to couple the first accessory part 2a', 2b' and the second accessory part 2a", 2b". Preferably, the first coupling element 15 and the second coupling element 14 form a watertight or a water-resistant connection between the first accessory part 2a', 2b' and the second accessory part 2a", 2b". In the shown embodiment, a first connectable accessory part 2a', 2b' comprises a first protruding element 3. A second connectable accessory part 2a", 2b" comprises a second protruding element 13, preferably flange-shaped. In the shown embodiment, the second coupling element 14 is provided in the second protruding element 13. It is imaginable that the second coupling element 14 is located elsewhere on the second accessory part 2a", 2b", for example above the second protruding element 13. The shown coupling elements 14, 15 are screw threads. Other coupling mechanisms to couple the first 15 and the second coupling element 14 are imaginable. The two shown medical accessories 2a, 2b differ from one another in that the right medical accessory 2b comprises two open ends 19, 20. The left medical accessory 2a comprises at least one closed end, which is closed by the protruding element 3. The at least one closed end is configured to block fluid, in particular to block fluid from entering and/or from exiting a port of an endoscope upon connecting the system 1 to an endoscope. It is imaginable that a medical accessory comprises two closed ends. The right medical accessory 2b comprises two open ends 19, 20 for conducting fluid, in particular to conduct fluid to a port of an endoscope upon connecting the system 1 to an endoscope. The right medical accessory 2b further comprises a resistance element 18, such as a hose barb. The shown resistance element 18 is provided at the first end 19 of the medical accessory 2b. The resistance element 18 may be configured to be connected to a fluid tube for conducting fluid, in particular to conduct fluid to a port of an endoscope upon connecting the system 1 to an endoscope. For example, to connect a cleaning device to an endoscope via the medical accessory 2b. The right medical accessory 2b further comprises one annular recess 10a. The annular recess 10a is provided at the second end 20 of the medical accessory 2b. The annular recess 10a is configured for accommodating an O-ring 11. The left medical accessory 2a comprises two annular recesses 10a, 10b for accommodating two O-rings 11, 12. These O-rings 11, 12 are configured to provide separation between channels, such as a water channel and/or an air channel, in an endoscope.

Second, the system 1 comprises a slide 4 comprising at least one passage opening 7 for passaging at least one medical accessory 2a, 2b. The shown embodiment comprises two passage openings 7 each configured for passaging a medical accessory 2a, 2b. The passage openings 7 extend between an upper side 5 and a lower side 6 of the slide 4. The passage openings 7 each comprise a wider part 9 and a narrower part 8 at the upper side 5 of the slide 4. The wider part 9 is configured for receiving the medical accessory 2a, 2b in the passage opening 7 of the slide 4. The narrower part 8 is configured to limit movement of the medical accessory 2a, 2b with respect to the slide 4, in particular to limit movement of the medical accessory 2a, 2b in a transverse plane of the upper side 5 of the slide 4. The shape of the passage opening 7 at the upper side 5 of the slide 4 may be chosen such to provide smooth movement and/or to reduce the resistance during movement of the slide 4 relative to the medical accessory 2a, 2b between the narrower part 8 and the wider part 9 of the passage opening 7 or between a first position and a second position. In the shown embodiment, the passage opening 7 at the upper side 5 of the slide 4 is eight-shaped. However, other shapes comprising a wider part 9 and a narrower part 8 are imaginable. At the lower side 6 of the slide 4, the passage opening 7 has a tapered or asymmetric shape (not shown). In the shown embodiment, the passage opening 7 further comprises a bulging portion 21 at the lower side 6 of the slide 4. The shown bulging portion 21 extends inwardly from the perimeter of the passage opening 7 to the passage opening 7. The bulging portion 21 is configured to make contact with and/or to seize on at least a part of a port of an endoscope upon coupling the system 1 to a port of an endoscope. In particular, the bulging portion 21 is provided in a slimmer portion of the tapered or asymmetric shape of the passage opening 7 at the lower side 6 of the slide 4. The medical accessories 2a, 2b are configured to be inserted in their respective passage opening 7 of the slide 4. Preferably, the first accessory parts 2a', 2b' may each be inserted in the respective passage opening 7 at the upper side 5 of the slide 4, and the second accessory parts 2a", 2b" may each be inserted in the respective passage opening 7 at the lower side 6 of the slide 4. Preferably, such that the protruding element 3 is in contact with the upper side 5 of the slide 4. The accessory parts 2a', 2a"; 2b', 2b" may be coupled in the wider part 9 of the passage opening 7 at the upper side 5 of the slide of the slide 4. Preferably, the first accessory part 2a', 2b' and the second accessory part 2a", 2b" are coupled such that the protruding element 3 abuts the upper side 5 of the slide 4. It is imaginable, that the second protruding element 13 will be received in the passage opening 7 at the lower side 6 of the passage opening 7. Preferably, the first accessory part 2a', 2b' and the second accessory part 2a", 2b" are coupled in a broader portion of the passage opening 7 at the lower side 6 of the slide 4. The shown slide 4 further comprises a deformable space 16 to provide a smooth transition between a first position and a second position. The deformable space 16 is provided adjacent to at least a part of at least one passage opening 7. In the shown embodiment, the deformable space 16 is provided between the two adjacent passage openings 7. The shown deformable space 16 is in alignment with at least one long side of at least one passage opening 7. The deformable space 16 is at least partially located at the upper side 5 of the slide 4 to provide a smooth transition of the medical accessories 2a, 2b between the wider part 9 and the narrower part 8 of the passage opening 7 at the upper side 5 of the slide 4. It is imaginable that the deformable space 16 is (also) provided at the lower side 6 of the slide 4 to provide a smooth transition of the medical accessories 2a, 2b in the tapered or asymmetric shape of the passage opening 7 at the lower side 6 of the slide 4. The slide 4 further comprises a grip 17 or a gripping portion configured for gripping the slide 4. In the shown embodiment, the grip 17 has a curved shape.

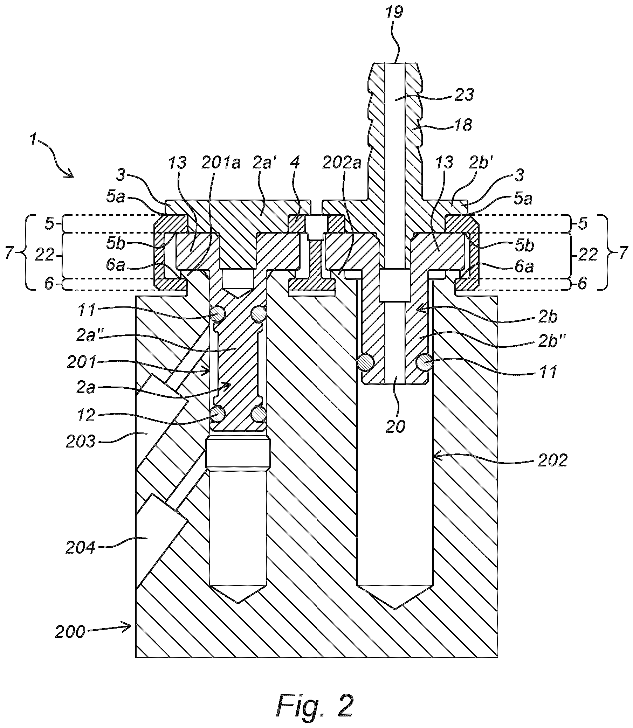

Figure 2 shows a cross-sectional view of the system 1 according to the invention, wherein the system 1 is connected to a part of an endoscope 200. The shown system 1 comprises two medical accessories 2a, 2b and a slide 4. The medical accessories 2a, 2b are inserted in a respective passage opening 7 of the slide 4. Both medical accessories 2a, 2b comprise a protruding element 3. The width of the passage opening 7 at an upper side 5 of the slide 4 is smaller than the width of the protruding element 3. The shown protruding element 3 is in contact with a part of the upper side 5 of the slide 4. In particular, the protruding element 3 is in contact with a top side 5a of the upper side 5 of the slide 4. The width of the passage opening 7 at the upper side 5 of the slide 4 is smaller than the width of the second protruding element 13. The shown second protruding element 13 is in contact with a part of the upper side 5 of the slide 4. In particular, the second protruding element 13 is in contact with a bottom side 5b of the upper side 5 of the slide 4. The first protruding element 3 and the second protruding element 13 of the medical accessory 2a, 2b are in contact, in particular within the passage opening 7 of the slide 4. The width of the passage opening 7 at the lower side 6 of the slide 4 is smaller than the width of the second protruding element 13. The right medical accessory 2b comprises a substantially hollow tube portion 23 configured to conduct fluid from the first (open) end 19 to the second (open) end 20 of the medical accessory 2b. The shown endoscope 200 is provided with two ports 201, 202, such as a suction valve, an air/water valve or an instrument channel. Both medical accessories 2a, 2b are at least partially provided in a respective port 201, 202 of the endoscope 200. In particular, the second accessory parts 2a", 2b" are provided in a respective port 201, 202 of the endoscope 200. The left port 201 of the endoscope 200 is connected or connectable to two channels 203, 204. The channels are for example an air channel 203 and a water channel 204. The left medical accessory 2a may be configured to separate the channels 203, 204. The shown left medical accessory 2a is provided with two O-rings 11, 12. The right medical accessory 2b is provided with one O-ring 11. A first O-ring 11 may be provided to limit, preferably block, fluid from escaping the port 201, 202 at the side of the slide 4. A second O-ring 12 may be provided to limit, preferably block, fluid from entering another channel 203, such as an air channel, than the channel 204 it originates from. Both shown ports 201, 202 comprise a flanged portion 201a, 202a. The second protruding element 13 of the medical accessories 2a, 2b is configured to be at least partially in contact with the flanged portion 201a, 202a of the ports 201, 202 of the endoscope 200. In particular, a lower side of the second protruding element 13 is in contact with the flanged portion 201a, 202a of the respective port 201, 202 of the endoscope 200, preferably the flanged portions 201a, 202a at least partially support the second protruding element 13. The slide 4 further comprises a lower side 6. The lower side 6 is located at the opposite side of the upper side 5 of the slide 4. At least a part of the port 201, 202, in particular at least a part of the flanged portion 201a, 202a, of the endoscope 200 is at least partially located in a respective passage opening 7 of the slide 4. The shown system 1 is provided in the second position, because a part of the port 201, 202 of the endoscope 200 is positioned in a slimmer portion of the passage opening 7 at the lower side 6 of the slide 4. This can be observed, since the lower side 6 of the slide 4 is at least partially in contact with at least a part of the respective port 201, 202 of the endoscope 200 to limit movement between the slide 4 and (the port 201, 202 of) the endoscope at the lower side 6 of the slide 4. In particular, the flanged portions 201a, 202a of the respective ports 201, 202 are in contact with the passage opening 7 at the lower side 6 of the slide 4. The flanged portion 201a, 202a of the shown embodiment is in contact with a top part 6a of the lower side 6 of the slide 4. In particular, the lower side 6 of the slide 4 is in contact with a lower side of the flanged portion 201a, 202a of the port 201, 202 of the endoscope 200 to limit movement between the slide 4 and the endoscope 200, preferably to limit movement in a direction perpendicular to a plane defined by the lower side 6 of the slide 4. The passage opening 7 may be divided in three subsections, comprising an upper side 5, a lower side 6 and an area 22 between the upper side 5 and the lower side 6 of the slide 4. The passage opening 7 in the area 22 may be wider than the passage opening 7 at the upper side 5 and/or at the lower side 6 of the slide 4 to provide manoeuvre space to move the slide 4 between the first position and the second position. Preferably, the height of the passage opening 7 in the area 22 can be adjusted to the height of the second protruding element 13 and/or the height of the port 201, 202 and/or the height of the flanged portion 201a, 202a.

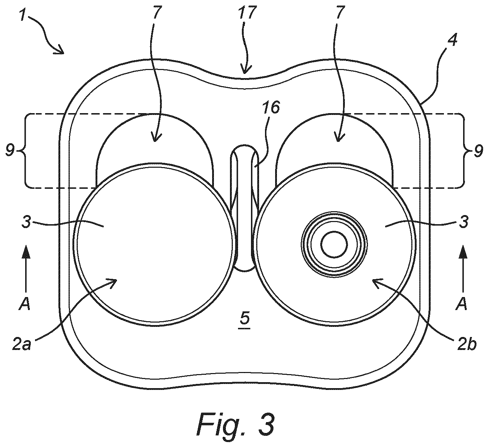

Figure 3 shows a top view of the system 1 according to the invention. The shown slide 4 comprises two passage openings 7 into which a medical accessory 2a, 2b is provided. The protruding elements 3 of the medical accessories 2a, 2b abut the upper side 5 of the slide 4. The slide 4 of the system 1 can be slid along the direction of the depicted arrows A, between the wider part 9 and the narrower part (not visible) of the passage opening 7 at the upper side 5 of the slide 4. In the first position, the medical accessories 2a, 2b are provided within the wider part 9 of the passage opening 7 at the upper side 5 of the slide 4. Whereas in the second position, the system 1 is arranged at least partially beyond the narrower part of the passage opening 7 at the upper side 5 of the slide 4. From the shape of the passage opening 7 from figure 1, it can be observed that the medical accessories 2a, 2b in figure 3 are positioned beyond the narrower part of the passage opening 7 at the upper side 5 of the slide 4. It is possible that at least a part of the narrower part (not visible) of the passage opening 7 is configured to be moved towards the deformable space 16 during the transition of the medical accessories 2a, 2b between the wider part 9 and the narrower part of the passage opening 7 at the upper side 5 of the slide 4.

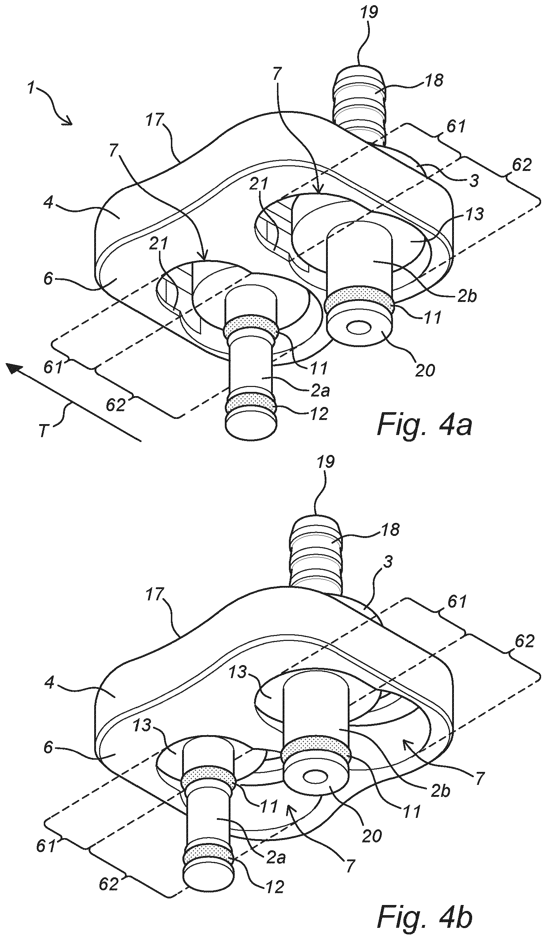

Figures 4a and 4b show a bottom view of an embodiment of the system 1 according to the present invention, wherein in figure 4a the slide 4 is provided in the first position, and wherein in figure 4b the slide 4 is provided in the second position. The passage opening 7 at the lower side 6 of the slide 4 has a tapered or asymmetric shape. The passage opening 7 tapers in the direction of the arrow T. The passage opening 7 at the lower side 6 of the slide 4 has a broader portion 62 and a slimmer portion 61. It is possible that the passage opening 7 at the lower side 6 of the slide 4 has a plurality of broader portions 62 and/or slimmer portions 61. The passage openings 7 of the shown slide 4 are pear-shaped, although other tapered or asymmetric shapes are imaginable. In the slimmer portion 61, the passage opening 7 comprises a bulging portion 21. The second protruding element 13 of the medical accessories 2a, 2b are entirely received in the corresponding passage opening 7 of the slide 4.

In the first position, as shown in figure 4a, both medical accessories 2a, 2b are provided in the broader portion 62 of the respective passage opening 7. In the first position, the medical accessory 2a, 2b may be placed in the passage opening 7 of the slide 4 and/or may be removed from the passage opening 7 of the slide 4. Additionally, in the first position, the medical accessory 2a, 2b may be at least partially be inserted or be placed in a port of an endoscope to be connected. Furthermore, in the first position, at least a part of the broader section 62 of the passage opening 7 at the lower side 6 of the slide 4 may be placed over and/or may be removed from a port of an endoscope to be connected to the system 1. After placing the medical accessory 2a, 2b in the first position or in the broader portion 62 of the passage opening 7, the slide 4 can be moved relatively to the medical accessory 2a, 2b. Preferably, such that the medical accessory 2a, 2b is moved towards the second position or slimmer portion 61. Furthermore, after placing the broader portion 62 of the passage opening 7 at the lower side 6 of the slide 4 over at least a part of a port, in particular a flanged portion of the port, of an endoscope to be connected or in the first position, the slide 4 can be moved relatively to the respective port of the endoscope.

In the second position, as shown in figure 4b, both medical accessories 2a, 2b are provided in the slimmer portion 61 of the respective passage opening 7 at the lower side 6 of the slide 4. In the second position, at least a part of a port of an endoscope to be connected is also provided in the slimmer portion 61 of the passage opening 7 at the lower side 6 of the slide 4. In particular, at least a part of a port of the endoscope to be connected is at least partially in contact with the bulging portion 21. In particular, the bulging portion 21 at least partially in contact with a flanged portion of a port of an endoscope to be connected. Preferably, such that the lower side of the second protruding element 13 is in contact with the respective port, in particular the flanged portion of the respective port, of the endoscope. In the second position, the medical accessory 2a, 2b may be locked in the passage opening 7 of the slide 4, preferably such that the medical accessory 2a, 2b may not be removed or released from the slide 4. Additionally, in the second position, the slide 4 may be locked to a port, in particular a flanged portion of the port, of an endoscope to limit movement between the slide 4 and the port of the endoscope. At a desired moment, the slide 4 can be moved relatively to the medical accessory 2a, 2b. The slide 4 can be moved relatively to the medical accessory 2a, 2b such that the medical accessory 2a, 2b, and hence the port of the endoscope, is moved towards the first position or broader portion 62 of the passage opening 7 at the lower side 6 of the slide 4, for example to disconnect the system 1 from the endoscope.

The above described figures are meant for illustrative purposes and are not limitative to the invention as claimed in the following claims. It is conceivable that individual inventive concepts may be applied without, in so doing, also applying other details of the described example. It is not necessary to elaborate on examples of all conceivable combinations of the above-described inventive concepts, as a person skilled in the art will understand numerous inventive concepts can be (re)combined in order to arrive at a specific application.

A person skilled in the art would understand that with an endoscope also a bronchoscope, colonoscope, laryngoscope, gastroscope, laparoscope, cystoscope, and the like, can be meant.

It will be apparent that the invention is not limited to the working examples shown and described herein, but that numerous variants are possible within the scope of the attached claims that will be obvious to a person skilled in the art.

The verb "comprise" and conjugations thereof used in this patent publication are understood to mean not only "comprise", but are also understood to mean the phrases "contain", "substantially consist of", "formed by" and conjugations thereof.

## Claims

1. System for connecting a medical accessory to an endoscope, comprising:
- the medical accessory comprising at least one protruding element, and
- a slide, comprising:
o an upper side and a lower side, and
o at least one passage opening extending between the upper side and the lower side of the slide configured for passaging at least a part of the medical accessory,
- wherein the slide is movable, relative to the medical accessory between a first position and second position,
- wherein in the first position,
o the medical accessory is positioned in the wider part of the at least one passage opening at the upper side of the slide, and
o at least a part of the endoscope to be connected to the medical accessory is positioned in a broader portion of the at least one passage opening at the lower side of the slide, and
- wherein in the second position,
o the medical accessory is positioned at least partially beyond the narrower part of the passage opening at the upper side of the slide, and
o at least a part the endoscope is positioned in a slimmer portion of the at least one passage opening at the lower side of the slide to limit movement between the slide and the endoscope.

2. System according to claim 1, wherein a width of the at least one passage opening at the upper side of the slide is smaller than a width of the at least one protruding element of the medical accessory to limit movement between the slide and the medical accessory,
wherein at the lower side of the slide the at least one passage opening has a tapered or asymmetric shape and/or
wherein at the upper side of the slide the at least one passage opening comprises a wider part and a narrower part.

3. System according to claim 1, wherein the at least one passage opening at the lower side of the slide at least partially tapers along a slide direction, wherein the slide direction extends between the first position and the second position of the slide.

4. System according to one of the preceding claims, wherein the wider part of the at least one passage opening at the upper side of the slide at least partially aligns with at least a part of the broader portion of the tapered shape or asymmetric shape of the at least one passage opening at the lower side of the slide to allow insertion of the medical accessory and/or to allow the insertion of at least a part of the endoscope in the passage opening of the slide.

5. System according to one of the preceding claims, wherein the medical accessory is configured to be connected to and/or to be inserted in at least one port of the endoscope, the port for example being a suction valve, an air/water valve or an instrument channel of the endoscope.

6. System according to one of claim 5, wherein the width of the slimmer portion of the at least one passage opening of the slide at the lower side of the slide is smaller than a flanged portion of the at least one port of the endoscope.

7. System according to one of the preceding claims, wherein the width of the at least one passage opening at the upper side of the slide comprises a narrowest part.

8. System according to claim 7, wherein the narrowest part is arranged between two side ends of the at least one passage opening.

9. System according to one of the preceding claims, wherein the medical accessory is substantially oblong, wherein the at least one protruding element at least partially protrudes in a substantially perpendicular direction to a longitudinal direction of the medical accessory,
wherein the at least one protruding element is configured to be at least partially in contact with and/or to at least partially abut the upper side of the slide, and/or
wherein the medical accessory comprises at least one second protruding element located below the at least one protruding element, preferably wherein the second protruding element is configured to be at least partially in contact with and/or to at least partially abut the lower side of the slide to limit movement between the slide and the medical accessory.

10. System according to one of the preceding claims, wherein the medical accessory comprises at least two ends located on opposite sides of the medical accessory.

11. System according to one of the preceding claims, wherein the medical accessory comprises an annular recess at a lower side of the protruding element for accommodating an O-ring configured to provide a seal in a space between at least a part of the medical accessory to be connected to at least a part of the endoscope and the endoscope and/or
wherein the medical accessory comprises at least two annular recesses at a lower side of the protruding element for accommodating two O-rings configured to provide separation between channels, such as a water channel and/or an air channel, in an endoscope.

12. System according to one of the preceding claims, wherein the slide is arranged to move, more in particular to slide, in direction perpendicular relative to a longitudinal direction of the medical accessory,
wherein the slide comprises a grip or a gripping portion for gripping the slide and exerting a force on the slide,
wherein the slide is at least partially composed of a polymer material, in particular a thermoplastic polymer material, such as polypropylene or polyamide and/or
wherein the medical accessory is at least partially composed of a metal, in particular of steel, more in particular of stainless steel.

13. Method for connecting a medical accessory to an endoscope, the method comprising the steps of:
a) providing a medical accessory comprising at least one protruding element,
b) providing a slide, comprising:
o an upper side and a lower side, and
o at least one passage opening extending between the upper side and the lower side of the slide configured for passaging at least a part of the medical accessory,
c) passaging the medical accessory at least partially through a wider part of the at least one passage opening at the upper side of the slide,
d) passaging at least a part of a port of an endoscope at least partially through a broader portion of the at least one passage opening at the lower side of the slide,
e) moving the slide relative to the medical accessory and/or the port of the endoscope, such that the medical accessory is positioned at least partially beyond the narrower part of the passage opening at the upper side of the slide and such that at least a part of the port of the endoscope is positioned in a slimmer portion of the at least one passage opening at the lower side of the slide.

14. Method for connecting a medical accessory to an endoscope according to claim 13, wherein a width of the at least one passage opening at the upper side of the slide is smaller than a width of the at least one protruding element of the medical accessory to limit movement between the slide and the medical accessory,
wherein at the lower side of the slide the at least one passage opening has a tapered or asymmetric shape and/or
wherein at the upper side of the slide the at least one passage opening comprises a wider part and a narrower part.
